(19) Europäisches Patentamt
European Patent Office
Office européen des brevets

(11) **EP 1 574 570 A1**

(12) **EUROPEAN PATENT APPLICATION**

(43) Date of publication:
**14.09.2005 Bulletin 2005/37**

(51) Int Cl.$^7$: **C12N 15/10**

(21) Application number: **04005913.1**

(22) Date of filing: **12.03.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL LT LV MK**<br><br>(71) Applicant: **Universität Regensburg**<br>**93040 Regensburg (DE)** | (72) Inventors:<br>• **Fuhrmann, Markus**<br>**93158 Teublitz (DE)**<br>• **Hegemann, Peter**<br>**93092 Friesheim (DE)**<br><br>(74) Representative: **Grünecker, Kinkeldey, Stockmair & Schwanhäusser Anwaltssozietät**<br>**Maximilianstrasse 58**<br>**80538 München (DE)** |

(54) **Process for reducing the number of mismatches in double stranded polynucleotides**

(57)  The present invention relates to a process for reducing the number of mismatches in a population of double stranded polynucleotides. Furthermore, the present invention relates to a process of producing double stranded polynucleotides. Also, the invention is directed to the use of an enzymes that cleaves and/or removes cleaved mismatches in double stranded polynucleotides in a process according to the invention as well as a kit of parts.

Fig. 1

principle of mismatch removal step:

mismatch cleaving enzyme T4 Endonuclease VII

melting of short overhangs at 37°C generates single stranded overhangs

single strand specific exonuclease (E. coli Exonuclease I)

**Description**

**Field of the invention**

The present invention relates to a process for reducing the number of mismatches in a population of double stranded polynucleotides. Furthermore, the present invention relates to a process of producing double stranded polynucleotides. Also, the invention is directed to the use of an enzyme that cleaves and/or removes cleaved mismatches in double stranded polynucleotides in a process according to the invention as well as a kit of parts, comprising at least one enzyme for cleaving in the vicinity of mismatches of double stranded polynucleotides, and at least one enzyme for enzymatic removal of the resulting 5' or 3' single stranded overhangs in the vicinity of the mismatching nucleotides in the originally double stranded polynucleotides.

**Background of the invention**

Present methods for the *in vitro* synthesis of double stranded polynucleotides (DNA and RNA) generally use chemically synthesized oligonucleotides, that are ligated or complemented by enzymes. After arranging the oligonucleotides of the forward strand continuously or discontinuously to overlap with the oligonucleotides of the reverse strand, single or double stranded polynucleotides are formed either by direct ligation or complementation of the gaps. The result of the synthesis strategy depends strongly on the quality of the starting oligonucleotide compounds. If, at an average length of 40 nucleotides in each oligonucleotide, 99% of these have the correct sequence and 1% have a wrong one, then a typical end product of a 1,000 nucleotides in length (25 oligonucleotide segments) has a 77% probability to have the correct sequence. If the quality is lowered by only 1 % to 98% correct oligonucleotides, then the product quality is already lowered to 60% correct products. With present procedures and the best available quality of oligonucleotides, about 10 to 20% "correct" products are obtainable. In conclusion, there must be an oligonucleotide quality of not less than 92 to 94% correct oligonucleotides available, when using, for example, the method for single-stranded synthesis strategy according to Hegemann (US patent 6,472,184 B1) or the double-strand synthesis strategy according to Sutton et al. (Sutton, D. W. (1995), Epicentre Forum 2 (2), 1).
Examples of present methods for gene synthesis are:

"Direct cloning": Two completely synthetic oligonucleotides are hybridized to each other. The length of the products is limited to about 150 to 200 base pairs due to technical limitations for the chemical synthesis of the oligonucleotides. Such a DNA has a high error probability since the error frequency in oligonucleotides increases considerably when synthesizing oligonucleotides having more than 40 to 45 nucleotides in length.

"Fill in synthesis": Here, only partially overlapping long oligonucleotides are hybridized to each other and the remaining single-stranded segments are filled by polymerases. In this method the length of the products is also limited by the maximum length of the used oligonucleotides. The product can have at most a length of two times the length of the originally used oligonucleotide, i.e. 300 to 400 base pairs.

"Double-strand synthesis" (for example, according to Sutton et al., (1995) Epicentre Forum 2 (2), 1): This method requires the ligation of several partially overlapping oligonucleotides of 40 to 50 bases in length with an overlapping region of 10 to 25 bases each. The oligonucleotides are chemically synthesized; prematurely terminated products are removed using the NENsorb-System according to the instructions of the manufacturer (NEN-DuPont), HPLC purification, or electrophoretic PAGE purification. The oligonucleotides are mixed in equimolar amounts and then phosphorylated at the 5'-end using a polynucleotide kinase and ATP. These oligonucleotides are specifically linked together, preferably with a thermostable ligase at high temperatures. The product produced in this manner is then ligated directly to a suitable vector DNA by adding a further mesophile DNA-ligase and ATP in a concentration of 5 to 10 pmol and cloned into host cells, for example *E. coli*. Alternatively, the product of the ligation reaction can first be amplified by polymerase chain reaction and then be cloned into suitable vector DNAs according to standard procedures.

"Single-stranded synthesis" (for example, according to Hegemann (US patent 6,472,184 B1): This method is similar to the double-stranded synthesis described above, however, only the oligonucleotides forming one of the polynucleotide strands constituting the double strands are phosphorylated at the 5'-end using polynucleotide kinase. Therefore, in the following ligation, only one strand consists of synthetically produced nucleic acids. The second strand is prepared later, for example, by a PCR reaction. The advantage of this method is, that the sequence and also the mistakes therein are only present in a single synthetic strand of the double-stranded end product. Errors in the oligonucleotides of the second chain do not manifest themselves in the end product. The cloning of these products is also done according to standard procedures.

"Sloning method" (for example, according to patent DE 19925862 A1, WO 0075368): This method is based on the ligation of very short oligonucleotides that leads to an extension of the product by only four base pairs for each reaction cycle. The intermediate products of each reaction cycle are then cleaved by restriction endonucleases and thereby activated for the next cycle. For a product of 1,000 base pairs a total of 250 reaction cycles must be performed, however, these may be performed in parallel so that intermediate products of about 20 to 30 base pairs are generally produced that are subsequently ligated to each other. An advantage of this method is that with a limited number of small oligonucleotides most desirable end products may be produced. Its disadvantage is a high consumption of enzymes, the insufficient efficiency of the individual ligation steps at low temperatures and the high number of individual steps (mostly a sequential and only partially a parallel procedure).

For all presently known methods for synthetically producing double stranded polynucleotides, including those methods described above, the quality of the results is directly and exponentially dependent on the employed oligonucleotides. Because of this high dependency, the employed oligonucleotides require high efforts for their synthesis, purification and quality control. The employed oligonucleotides must be prepared individually and the by-products are generally removed laboriously, e.g. by HPLC or PAGE. Furthermore, the final double stranded polynucleotide end products must be completely analyzed by sequencing. The "Sloning" method requires less effort with respect to oligonucleotide quality control due to the versatile small-sized oligonucleotide building blocks. But this advantage is compensated by the consumption of large amounts of expensive enzymes.

There are a number of compounds and methods available for detecting mismatches in heteroduplex polynucleotides. For example, so-called "resolvases" have been used for mismatch detection techniques for identifying or evaluating mutations in nucleic acid sequences, in particular to identify heritable changes in genetic material.

PCT WO 99/40222 relates to the use of the resolvase endonuclease VII of bacteriophage T4 (T4 EndoVII) (Golz et al., DNA Res. 2:277-284 (195); Kemper et al., Eur. J. Biochem. 115:123-131 (1981 )) which detects all possible mismatches including C/C mismatches, heteroduplex loops, single nucleotide bulges, single-stranded overhangs, branched DNAs, bulky adducts, psoralen crosslinks and apurinic sites. The broad substrate specificity makes the enzyme an extremely versatile tool for mismatch detection (Cotton, Mutation Detection, Oxford University Press, Oxford (1997). The nucleolytic activity of T4 EndoVII has been used successfully to detect mutations in heteroduplex DNA by cleavage assays.

Besides T4 EndoVII, the resolvases include, among others, bacteriophage T7 endonuclease I (West, Ann, Rev. Biochem. 61, 603 (1992)), *E. coli* endonuclease V (Yao M, Kow YW, J. Biol. Chem. 272, 30774 (1997)); and eukaryotic resolvases, particularly those from the yeast *S. cerevisiae,* which have been shown to recognize and cleave cruciform DNA (West, supra; Jensch et al., EMBO, J. 8, 4325 (1989)); S1 nuclease which is also capable of recognizing and cleaving DNA mismatches (Shenk et al., Proc. Natl. Acad. Sci. 72, 989 (1975)); and the Mut Y repair protein of *E. coli* (Lu et al., Genomics 14, 249, (1992)).

In summary, mismatches from synthetic strategies aimed at producing double stranded polynucleotides are a nuisance that requires cost and work intensive purification as well as the use of high quality and expensive starting compounds. Therefore, there is a strong need for improving the quality of synthetically produced double stranded polynucleotides. In particular, there is a need for avoiding the high effort and high costs involved in the synthesis, purification and quality control of the oligonucleotides employed for double stranded polynucleotide synthesis. Furthermore, it is desirable to lower the fraction of mismatched double stranded polynucleotides in ligation products.

**Disclosure of the invention**

The present invention provides in one aspect a process for reducing the number of mismatches in a population of double stranded polynucleotides, comprising the steps of:

a) cleaving the double-stranded polynucleotide in the vicinity of a mismatch enzymatically, then
b) removing the previously mismatching nucleotides from both strands of the double-stranded polynucleotide enzymatically,
c) filling the resulting gaps by separating the strands and allowing them to anneal to form overlapping templates, followed by enzymatic polymerization for completing a double strand with filled gaps.

The term PCR as used herein also includes overlap-extension PCR (as described in, e.g., Aiyar A, Xiang Y, Leis J. (1996). Methods Mol Biol. 57:177-91).

The term "removal" of cleaved mismatches is meant to comprise enzymatic removal accomplished by nucleolytic action of an enzyme.

In order to increase the number of available polynucleotides, the whole population may be subjected to any known amplification procedure, preferably subsequent to mismatch correction. A preferred method for amplifying is polymerase chain reaction (PCR).

The present invention is applicable to DNA and RNA polynucleotides as well as to natural and synthetic derivatives of RNA and DNA that can be recognized and cleaved by enzymes. The preferred embodiment of the process of the present invention relates to reducing mismatches in double stranded DNA.

The term "mismatches" in double stranded polynucleotide as used herein is understood to relate to double-stranded polynucleotides, in which there is one or more nucleotide in one strand that does not pair through Watson-Crick base pairing and π-stacking interactions with a nucleotide in its complementary strand. This type of disturbance is often caused by a change in one or more nucleotides, e.g. chemical changes in the base portion of the nucleotide. Disturbances or modifications of this kind are recognized by mismatch-binding proteins. Exemplary base modifications are replacements, deletions, insertions of bases, presence of chemically altered bases, apurinic and apyrimidinic sites, and the presence of protection groups resulting from a chemical oligonucleotide synthesis process, etc.

According to the present invention, the region(s) of the polynucleotides that comprise(s) a mismatch is (are) specifically bound by an enzyme that cleaves the polynucleotide in the vicinity of said mismatch. The exact location of the cleavage on one or both strands depends on the specific enzyme used.

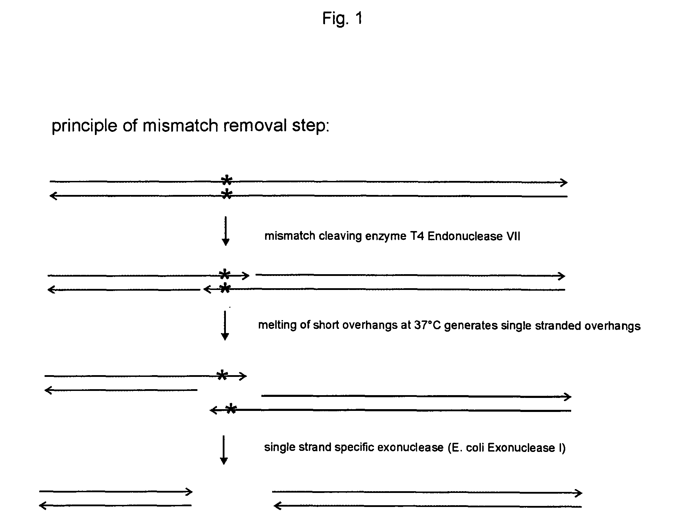

For example, in a first step a) T4 Endo VII cuts asymmetrically on both strands 3' from the mismatch site at a variable distance of between 1 and 6 bases, *E. coli* Endo V cuts both strands at a distance of 2 or 3 bases 3' from the mismatch. The resulting overlaps between the nicks are short (<10 bp) and lack complete base pairing due to the mismatches. As a consequence, they tend to dissociate at ambient temperature resulting in two double stranded fragments with sticky ends. In a further step b) the single stranded overhangs are removed by an enzyme with exonuclease activity, preferably specific for single stranded polynucleotides. See Fig. 1 for scheme.

For step a) enzymes that specifically cleave in the vicinity of the mismatch(es) of double stranded polynucleotides are used. Endonucleases with the necessary enzymatic specificity are known to those skilled in the art. Examples are T4 Endo VII, T7 Endo I, *E. coli* Endo V, *Aspergillus oryzae* S1 nuclease, *Ustilago maydis* DNAseI, *E. coli* Mut Y, *E. coli* Exonuclease I *and Vent-DNA-polymerase.*

The "vicinity" of mismatches is generally considered to comprise 25, preferably 15, more preferably 10, most preferably 5 nucleotides next to the mismatch.

For removal of the cleaved mismatched regions in step b) any second enzyme capable of specifically degrading protruding single stranded polynucleotides can be applied. Examples thereof are *E. coli* exonuclease I, *E. coli* DNA polymerase I (and the Klenow. fragment thereof), T4 DNA polymerase, Vent DNA polymerase, Pfu DNA polymerase; mung bean nuclease, etc.

The parameters for the enzymatic reactions of steps a) to c) depend on the particular oligonucleotides and enzymes employed. Among these parameters are the specific enzymes used and incubation conditions, e.g. incubation time, temperature, buffer(s), pH, auxiliary subtances, such as e.g. detergents, DTT, etc., for the enzymatic reactions of steps a) to c) depend on the particular oligonucleotides and enzymes employed. Generally, the state of the art will provide suitable information and incubation conditions, so that an optimization for performing the specific enzymatic reactions is within the ordinary skill of the expert in the field of biotechnology and does not go beyond routine experimentation. Generally, acting in compliance with the instructions of the manufacturer should provide the desired result.

Preferably, the enzymes used in step a) and/or b) are selected from the group consisting of selected from the group consisting of T4 Endo VII, T7 Endo I, *E. coli* Endo V, *Aspergillus oryzae* S1 nuclease, *Ustilago maydis* DNAseI, *E. coli* Mut Y, *E. coli* Exonuclease I, *Vent-DNA-polymerase,* Pfu DNA polymerase, and mung bean nuclease.

In the following, further information is provided for a limited number of selected preferred enzymes. It is to be understood that this specific information is by no means limiting but merely serves illustrative purposes.

T7 phage endonuclease I (the product of the T7 phage gene 3) New England Biolabs, Cat. # M0292S or M0292L,

T4 phage endonuclease VII: Golz, Birkenbihl, Kemper 1995 DNA research 2, 277-284.

*E. coli* endonuclease V: *E. coli* product of gene nfi, orf 225, Trevigen, Order no. 4035-500-EB or 4035-500-K, described in Guo, G., Y. Ding, B. Weiss 1997. nfi, the gene for endonuclease V in *Escherichia coli* K-12. J. Bacteriol. 179:310-316

DNAse I from Ustilago: described in Ahmad, Holloman, Holliday 1975 Nature 258, 54-56.

*E. coli* exonuclease I: New England Biolabs, Cat. # M0293S, Lehman, Nussbaum 1964, JBC 239, 2628

*Thermococcus litoralis* Vent-DNA polymerase: New England Biolabs, Cat. # M0254S Mattila et al 1991, Nucl. Ac.

Res. 19, 4967-73.

*Aspergillus oryzae* S1 nuclease: MBI Fermentas, Cat. # EN0321, Vogt 1973, Eur. J. Biochem. 33, 192-200.

In a more preferred embodiment, the enzyme in step a) is T4 Phage Endonuclease VII and the enzyme in step b) is *E. coli* Exonuclease I.

Also preferred is that the polymerase used in step b) and/or step c) is a proofreading polymerase.

Most preferably, the polymerase used in step b) or step c) is Vent-DNA-Polymerase.

In another most preferred embodiment of the invention the enzyme used in step b) and step c) is Vent-DNA-Polymerase.

In a further most preferred embodiment of the invention the polymerase used in step a) is T4 Phage Endonuclease VII, the enzyme used in step b) and step c) is Vent-DNA-Polymerase.

In a further aspect the present invention also relates to processes for producing double stranded polynucleotides having a reduced number of mismatches in their double strands, comprising the steps of:

a) providing $n$ 5'-phosphorylated linkable oligonucleotides designed to form a first strand and $m$ oligonucleotide(s) corresponding to the complementary strand, wherein each oligonucleotide of the complementary strand overlaps with at least two oligonucleotides of the first strand, wherein $n$ is an integer from 2 to 100 and $m$ is between $n$ -1 and $n$ + 1.

b) allowing the oligonucleotides of the first strand to anneal to those of the second strand

c) linking the oligonucleotides of both strands to produce a double-stranded polynucleotide

d) reducing the number of mismatches in the population by subjecting the double-stranded polynucleotide of step c) to a process according to any one of claims 1-11.

In the above process the steps a) to c) preferably lead to "perfectly" complementary double stranded polynucleotides. In reality, there is mostly a proportion of double stranded polynucleotides comprising mismatches. The number of these mismatches then can be reduced by step d), i.e. by subjecting the double stranded polynucleotides to a process as described in detail above.

Preferably, steps a) to c) relate to a polynucleotide synthesis strategy according to a method selected from the group consisting of "direct cloning," "fill-in synthesis," "double strand synthesis," "single strand synthesis," or the "Sloning" method. It is of course understood that limiting steps a) to c) to particular technical process features would lead to an undue limitation of the general scope of the invention. The person skilled in the art is well aware of many strategies and deviations thereof to produce polynucleotides by linking oligonucleotides. Therefore, these steps must be expressed in more general terms.

In another aspect, the present invention is directed to the use of enzymes that cleave and/or remove cleaved mismatches in double stranded polynucleotides in a process according to the invention.

Preferably, the enzyme is selected from the group consisting of endonuclease VII from T4 phage, endonuclease I from T7 phage, endonuclease V from *E. coli,* S1 nuclease from *Aspeigillus oryzae,* DNAsel from *Ustilago maydis, E. coli* Mut Y, Exonuclease I *from E. coli, Vent-DNA-polymerase,* Pfu DNA polymerase, and mung bean nuclease.

A further aspect of the invention relates to a kit of parts, comprising at least one enzyme for cleaving mismatches of double stranded polynucleotides, and at least one enzyme for enzymatic removal of the cleaved mismatches in the double stranded polynucleotides.

In a preferred embodiment said kit additionally comprises at least one enzyme having an activity for performing a PCR (polymerase chain reaction). Suitable enzymes for performing PCR are, e.g. Taq-DNA-polymerase (several suppliers, e.g. MBI, NEB, Stratagene, etc.), Vent-/DeepVent-DNA-polymerase (New England Biolabs), Pfu- (native/ cloned), Pfu-Turbo-, Pfu-Ultra-Polymerase (Stratagene), Herculase (Stratagene), Optimase DNA Polymerase Plus/ Premium/Ultra (Transgenomic Inc.), Phusion High-fidelity DNA-polymerase (MJ research), ISIS proofreading polymerase/Tfu DNA polymerase (Qbiogene), KOD HiFi/XL DNA polymerase (Novagen), Expand High fidelity (Roche Appl. Sci.), Elongase enzyme mix (Invitrogen), etc..

In a more preferred embodiment, said kit may, in addition, comprise instructions for performing the process according to the invention, and/or buffer solutions, vials, auxiliary substances, etc., that are useful for performing a process according to the invention.

**Figures**

Fig. 1 shows the principle of mismatch cleavage by T4 Endo VII and the removal of the resulting single stranded overhangs.

Fig. 2 summarizes the statistical evaluation of different gene synthesis methods for a codon adapted version of chloramphenicol acetyl transferase.

Analysis was performed as a combination of two consecutive assays of survival. To establish this simple test system the gene synthesis product was cloned between the unique restriction sites EcoRI and BamHI of the cloning vector pUC18. The synthetic gene was designed in a way that standard IPTG induced expression of the lacZ fragment of pUC18 leads to in frame translation of the synthetic gene.

The first test was done by transferring transformed (= ampicillin resistant) *E. coli* on LB-agar plates containing 100 μg/ml ampicillin and 0.1 mM IPTG (to induce lacZ expression). The number of surviving clones from this first test represents the "number of analyzed clones."

In a second survival test the ampicillin resistant clones from test 1 were transferred to LB-agar plates containing 100 μg/ml ampicillin, 0.1 mM IPTG and 34 μg/ml chloramphenicol. "Correct" clones are those that are able to grow on 34 μg/ml chloramphenicol containing plates.

The term "relative frequency" describes the ratio of the number of "correct" clones (chloramphenicol resistant) and the "number of analyzed clones" (ampicillin resistant). This value does not take into account the efficiency of the individual ligation and cloning step.

$$\text{Relative frequency} = \frac{\text{number of correct clones (chloramphenicol resistant)}}{\text{number of analyzed clones (ampicillin resistant)}}$$

The fraction of clones harboring no synthetic polynucleotide is variable from experiment to experiment and is not accounted for in this value. This uncertainty leads to the definition of a normalizing factor $\lambda$:

$$\lambda = \frac{\text{number of clones with 700bp insert}}{\text{number of analyzed clones (ampicillin resistant)}}$$

Multiplication of the "relative frequency" with $1/\lambda$ leads to the normalized value of the "relative frequency CAMr/ CAT", i.e., the ratio of "correct" clones (chloramphenicol resistant) and "total" clones harboring synthesis products of correct size (ca. 700bp).

$$\text{Relative frequency (CAMr/CAT)} = \frac{1}{\lambda} \text{ relative frequency}$$

$$= \frac{\text{number of correct clones (chloramphenicol resistant)}}{\text{number of clones with 700bp insert}}$$

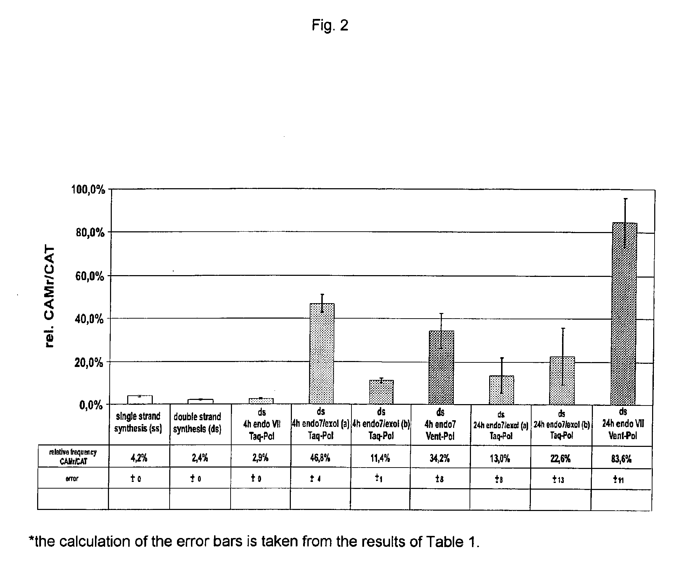

Figure 2 summarizes 9 different experiments, as listed in Table 1 below, with modifications of the gene synthesis and error correction procedure.

Table 1

| method of gene synthesis and correction conditions | number of analyzed clones | number of correct clones | relative frequency | $\lambda$ | relative frequency CAMr/ CAT |
|---|---|---|---|---|---|
| (1) ss synthesis no correction | 49 | 2 | 0,041 | 0,97 ±0,03 | 0,042 ± 0,00 |
| (2) ds synthesis no correction | 152 | 2 | 0,013 | 0,55 ± 0,01 | 0.024 ± 0,00 |
| (3) ds synthesis 4h EndoVII Taq amplification | 71 | 2 | 0,028 | 0,97 ± 0,03 | 0,029 ± 0,00 |

Table 1   (continued)

| method of gene synthesis and correction conditions | number of analyzed clones | number of correct clones | relative frequency | λ | relative frequency CAMr/ CAT |
|---|---|---|---|---|---|
| (4) ds synthesis 4h EndoVII+ExoI (Endo buffer) Taq amplification | 76 | 21 | 0,276 | 0,59 ± 0,05 | 0,468 ± 0,04 |
| (5) ds synthesis 4h Endo VII+ExoI (ExoI buffer) Taq amplification | 76 | 8 | 0,105 | 0,92 ± 0,08 | 0,114 ± 0,01 |
| (6) ds synthesis 4h Endo VII Vent amplification | 152 | 26 | 0,171 | 0,5 ± 0,11 | 0,342 ± 0,08 |
| (7) ds synthesis 24h EndoVII + ExoI (Endo buffer) Taq amplification | 76 | 1 | 0,013 | 0,10 ± 0,06 | 0,130 ± 0.08 |
| (8) ds synthesis 24h EndoVII + ExoI (Exo buffer) Taq amplification | 76 | 6 | 0,079 | 0,35 ± 0,20 | 0,226 ± 0,13 |
| (9) ds synthesis 24h Endo VII Vent amplification | 76 | 14 | 0,184 | 0,22 ± 0,03 | 0,836 ± 0,11 |

Column 1 of Fig. 2 shows the results of the single-stranded synthesis of example 3 without correction.

Column 2 shows the results for the double-strand synthesis of example 3 without correction.

Column C1 (C = correction) shows the results of the method used in columns 2, followed by four hours treatment with T4 Endo VII in T4 Endo VII buffer, followed by Taq-PCR.

Column C2 shows the result of the process according to C1 to which exonuclease 1 was added.

Column C3 shows the result of the process according to C2, however, incubation buffer for exonuclease 1 was used.

Column C4 shows the result of C1, except that Vent-DNA polymerase was used as exonuclease.

Column C5 shows the result of the process according to C2, wherein the incubation period was extended to 24 hours instead of 4 hours.

Column C6 shows the results of the process according to C3, wherein the incubation period was extended to 24 hours instead of 4 hours.

Column C7 shows the results of the process according to C4, wherein the incubation period was extended to 24 hours instead of 4 hours.

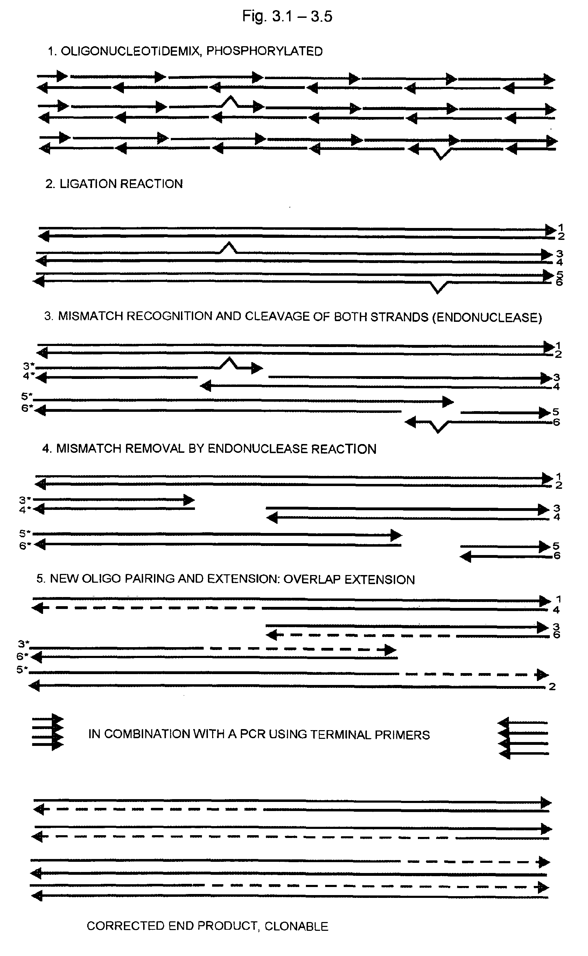

Fig. 3 shows the strategy according to the invention for producing oligonucleotides by ligation of oligonucleotides and correcting the resulting double stranded polynucleotides according to the invention.

Fig. 3.1 shows the alignment of phosphorylated oligonucleotides in a ligation mixture, wherein mismatches are depicted by kinks in the arrows.

Fig. 3.2 shows the result of the ligation reaction, including the mismatches.

Fig. 3.3 shows the result of enzymatic mismatch recognition and cleavage in the vicinity of mismatched regions.

Fig. 3.4 shows the removal of mismatched regions by exonuclease treatment.

Fig. 3.5 shows new strand pairing and extension along the overlapping ends combined with a PCR with added terminal primers resulting in corrected and clonable double stranded polynucleotides.

The following examples further illustrate the best mode contemplated by the inventors for carrying out their invention. The examples relate to preferred embodiments and are not to be construed to be limiting on the scope of the invention.

**Examples**

Mismatch detection - assay system

For mismatch detection a bacterial gene (chloroamphenicol acetyltransferse (CAT) is selected that leads to antibiotic resistance against chloroamphenicol. This gene serves as a test system for the ligation-based gene synthesis strategy of polynucleotides of medium quality. A successful synthesis is one, wherein the synthesis product is capable of eliciting chloroamphenicol resistance in the chosen host cell *E. coli.* This system does not detect point mutations that only lead to exchange of a position of the gene but that do not effect its function. These "mistakes" can only be identified by the complete synthesis of the products, which would not be practical for statistically evaluating the results.

Oligonucleotides and enzymes:

17 oligonucleotides (sense) with a length of 18 to 45 nucleotides (for sequences see table 1) of medium quality.

17 nucleotides (antisense) with a length of 30 to 45 nucleotides (for sequences see table 2) of medium quality.

2 oligonucleotides (terminal) with a length of 30/32 nucleotides (for sequences see table 3) of basic quality.

T4 polynucleotide kinase (NEB): 10 u/μl; *Taq*-DNA ligase (NEB): 40 u/μ l; Vent-DNA-polymerase; *Taq*-DNA-polymerase; Exonuclease I (*E. coli* NM554); T4 endonuclease VII (USB); T4 endonuclease VII (KFB); T7 endonuclease I.

Example 1: Oligonucleotide mix

Sense-mix: 10 μl of each sense oligonucleotide (c = 50 pmol/μl) is added to a vial and mixed (here total volume V = 170 μl).

Antisense-mix: 10 μl of each antisense oligonucleotide (c = 50 pmol/μl) is added to a vial and mixed (total volume V = 170 μl).

Example 2: Polynucleotide kinase reaction

PNK sense: 10 μl sense mix was phosphorylated with 20 units PNK for 3h at 37 °C. (Buffer: 1X T4 polynucleotide kinase reaction buffer [70 mM Tris-HCl (pH 7.6), 10 mM $MgCl2_2$, 5 mM dithiothreitol] and 2.5 mM ATP (total volume 100 μl)).

PNK antisense: 10 μl antisense-mix were phosphorylated with 20 units PNK for 3h at 37 °C (Buffer: 1X T4 polynucleotide kinase reaction buffer [70 mM Tris-HCl (pH 7.6), 10 mM $MgCl_2$, 5 mM dithiothreitol] and 2.5 mM ATP (total volume 100 μl)).

Example 3: Synthesis strategy:

a) Single-strand synthesis:

10 μl PNK sense, 1 μl antisense-mix were denatured in 1 X *Taq*-DNA ligase buffer:
[20 mM Tris-HCl (pH 7.6 at 25 °C), 25 mM potassium acetate, 10 mM magnesium acetate, 10 mM DTT, and 1 mM NAD, and 0.1% Triton X-100], for example 60 seconds at 95 °C, then cooled to 80 °C.

Addition of 60 U *Taq*-DNA ligase to start the synthesis reaction:

1 minute at 95 °C denatured all oligonucleotides in the mix.

6 minutes at 70 °C, then cooling with -0.01 °C /s to 65 °C, 6 minutes at 65 °C, then cooling with -0.01 °C/s to 60 °C, 6 minutes at 60 °C, then cooling with -0.01 °C/s to 56 °C. (Alternatively, the cooling may be done linearly or stepwise with other time periods and temperatures).

Repetition: 0 to 5 times, here 5 times.

Subsequent cooling to 4 °C results in a single-stranded crude ligation product.

b) Double-strand synthesis:

10 µl PNK sense, 10 µl PNK antisense were denatured in 1X *Taq*-DNA ligase buffer:
[20 mM Tris-HCl (pH 7.6 at 25 °C), 25 mM potassium acetate, 10 mM magnesium acetate, 10 mM DTT, and 1 mM NAD, and 0.1% Triton X-100], for example, 60 seconds at 95 °C, then cooled down to 80 °C.

Addition of 60 U *Taq*-DNA ligase started the synthesis reaction:

1 minute at 95 °C denatured all oligonucleotides in the mix.

6 minutes at 70 °C, then cooling with -0.01 °C/s down to 65 °C, 6 minutes at 65 °C, then cooling with -0.01 °C/s down to 60 °C, 6 minutes at 60 °C, then cooling with -0.01 °C/s down to 56 °C. (Alternatively, the cooling may be done linearly or stepwise with different time periods and temperatures.)

Repetition: 0 to 5 times, here 5 times.

Cooling down to 4 °C resulted in a double-strand crude ligation product.

Example 4: Error correction

5 µl double-strand crude ligation product was treated with (several combinations of) endo- and exonucleases [total volume of 20 µl, end concentration of 5 mM Tris-HCl, pH 8.0, 1 mM $MgCl_2$, 1 mM DTT, 10 µg/ml BSA]:

a) (500 units) T4 endonuclease VII + (20 units) exonuclease I

b) (500 units) T4 endonuclease VII

for 4 hours at 37 °C, or for 24 hours at 37 °C.

Example 5: Amplification of the end products by PCR

a) With Vent-DNA polymerase: 5 µl of the mismatch correction mix from example 4, 50 pmol of each terminal oligonucleotide as primer, 5 µl dNTP mix, 1X ThermoPol buffer: [10 mM KCl, 20 mM Tris-HCl (pH 8.8 at 25 °C), 10 mM $(NH_4)_2SO_4$, 2 mM $MgSO_4$, 0.1 % Triton X-100] and 1.6 units Vent-DNA polymerase.]

b) With *Taq*-DNA polymerase: 5 µl of the error correction mix, 50 pmol of each terminal oligonucleotide as primer, 5 µl dNTP mix, 1X ThermoPol buffer: [10 mM KCl, 20 mM Tris-HCl (pH 8.8 at 25 °C), 10 mM $(NH_4)_2SO_4$, 2 mM $MgSO_4$, 0.1 % Triton X-1 00] and 1 units *Taq*-DNA polymerase.

Conditions according to standard PCR protocols were used. The complete PCR mixture is then purified from primers by standard procedures (Nucleospin, Macherey-Nagel) and the DNA is cloned via the terminal restriction cleavage sites EcoRI and BamHI into pUC18-DNA via the same cleavage sites. Early selection is done on LB-agar plates with ampicillin (100 μg/ml) and IPTG (0.5 mM) for induction of the expression by incubation at 37 °C for 18 hours. The clones prepared in this manner were transferred to fresh LB-agar plates with ampicillin (100 μg/ml), IPTG (0.5 mM) and chloroamphenicol 34 μg/ml and further incubated at 37 °C. Those clones that demonstrate distinct growth on the plates with chloroamphenicol are considered "correct".

The "correct" sense and antisense oligonucleotides as well as the terminal oligonucleotides for PCR amplification are shown below.

## Table 1: Sense oligonucleotide

FWD-oligos:

```
CATF01:   ccg  ctc  gag  atg  gcc  gag
CATF02:   aag  aag  atc  acc  ggc  tac  acc  acc  gtg  gac  atc  tcc  cag  tgg  ca
CATF03:   ccg  caa  gga  gca  ctt  cga  ggc  ctt  cca  gtc  cgt  cgc  cca  gtg
CATF04:   cac  cta  caa  cca  gac  cgt  gca  gct  gga  cat  cac  cgc  ctt  cct  g
CATF05:   aag  acc  gtg  aag  aag  aac  aag  cac  aag  ttc  tac  ccc  gcc  ttc  atc
CATF06:   cac  atc  ctg  gcc  cgc  ctg  atg  aac  gcc  cac  ccc  gag  ttc  cg
CATF07:   cat  ggc  cat  gaa  gga  cgg  cga  gct  ggt  gat  ctg  gga  ctc  cgt
CATF08:   cca  ccc  ctg  cta  cac  cgt  gtt  cca  cga  gca  gac  cga  gac  ctt
CATF09    ctc  ctc  cct  gtg  gtc  cga  gta  cca  cga  cga  ctt  ccg  cca  gtt
corr:
CATF10:   cct  gca  cat  cta  ctc  cca  gga  cgt  cgc  ctg  cta  cgg  cga  ga
CATF11:   acc  tgg  cct  act  tcc  cca  agg  gct  tca  tcg  aga  aca  tgt  tct  tcg
CATF12    tgt  ccg  cca  acc  cct  ggg  tgt  cct  tca  cct  cct  tcg  acc  tga  a
corr:
CATF13:   cgt  ggc  caa  cat  gga  caa  ctt  ctt  cgc  ccc  cgt  gtt  cac  cat  g
CATF14:   ggc  aag  tac  tac  acc  cag  ggc  gac  aag  gtg  ctg  atg  ccc  ctg
CATF15:   gcc  atc  cag  gtc  cac  cac  gcc  gtg  tgc  gac  ggc  ttc  cac  g
CATF16:   tcg  gcc  gca  tgc  tga  acg  agc  tgc  agc  agt  act  gcg  acg  agt
CATF17:   ggc  agg  gcg  gcg  ccc  tac  gta  tct  aag  gat  ccc  g
```

Table 2: Anti-sense oligonucleotide

RWD-oligos:

```
CATR01:  gtg  gtg  tag  ccg  gtg  atc  ttc  ttc  tcg  gcc  atc  tcg  agc  gg
CATR02:  gcc  tcg  aag  tgc  tcc  ttg  cgg  tgc  cac  tgg  gag  atg  tcc  acg
CATR03:  ctg  cac  ggt  ctg  gtt  gta  ggt  gca  ctg  ggc  gac  gga  ctg  gaa  g
CATR04:  tgc  ttg  ttc  ttc  ttc  acg  gtc  ttc  agg  aag  gcg  gtg  atg  tcc  ag
CATR05:  cat  cag  gcg  ggc  cag  gat  gtg  gat  gaa  ggc  ggg  gta  gaa  ctt  g
CATR06:  tcg  ccg  tcc  ttc  atg  gcc  atg  cgg  aac  tcg  ggg  tgg  gcg  tt
CATR07:  aac  acg  gtg  tag  cag  ggg  tgg  acg  gag  tcc  cag  atc  acc  agc
CATR08:  tac  tcg  gac  cac  agg  cag  gag  aag  gtc  tcg  gtc  tgc  tcg  tgg
CATR09:  tcc  tgg  gag  tag  atg  tgc  agg  aac  tgg  cgg  aag  tcg  tcg  tgg
CATR10:  cct  tgg  gga  agt  agg  cca  ggt  tct  cgc  cgt  agc  agg  cga  cg
CATR11:  aca  ccc  agg  ggt  tgg  cga  aca  cga  aga  aca  tgt  tct  cga  tga  agc
CATR12:  gaa  gtt  gtc  cat  gtt  ggc  cac  gtt  cag  gtc  gaa  gga  ggt  gaa  ga
CATR13:  gcc  ctg  ggt  gta  gta  ctt  gcc  cat  ggt  gaa  cac  ggg  ggc  gaa
CATR14:  gcg  tgg  tgg  acc  tgg  atg  gcc  agg  ggc  atc  agc  acc  ttg  tc
CATR15:  gct  cgt  tca  gca  tgc  ggc  cga  cgt  gga  agc  cgt  cgc  aca  cg
CATR16:  cgt  agg  gcg  ccg  ccc  tgc  cac  tcg  tcg  cag  tac  tgc  tgc  a
CATR17:  cgg  gat  cct  tag  gcg  ccg  ccc  tgc  cac  tcg
```

Table 3: terminal oligonucleotides for PCR amplification

```
CATF01  ccg   ctc   gag   atg   gcc   gag
CATR17: cgg   gat   cct   tag   gcg   ccg   ccc   tgc   cac
```

Results:

If the CAT-synthesis is performed without enzymatic binding/cleaving and removal of mismatches according to the invention (i.e. without mismatch correction), then the used oligonucleotides result in the following relative fractions of resistant "correct" clones:

Single-stranded synthesis according to Hegemann US 6,472,184 B1: 2 resistant clones, 49 clones tested, λ =0,97: 4.2% (compare diagram of Fig. 2, process for column 1).

**Modified** double-strand synthesis according to Sutton et al.: (In the original method the synthesis product is directly ligated after synthesis with a suitable vector DNA. In this example the double stranded synthesis product is amplified with Taq-DNA Polymerase, the purified product digested with EcoRI and BamHI and then cloned): (1-) 2 resistant clones, 152 clones tested λ = 0,55: 2.4% (compare diagram of Fig. 2, process for column 2).

As expected, the single-stranded synthesis was less prone to errors.

If the synthesis is followed by an enzymatic binding and cleavage step (a) of the mismatches in the heteroduplex polynucleotides (i.e. with enzymatic recognition and cleavage of mismatches, for example, by T4 endonuclease VII), then this results only in a small improvement for the double stranded synthesis to 2.8% "correct" clones (compare the diagram of Fig. 2, column 3; single-stranded results are not shown).

If the synthesis is followed by enzymatic binding to and cleavage (step a) as well as removal of the mismatches (step b) from the heteroduplex polynucleotides (i.e. enzymatic recognition of erroneous ligation products, for example by T4 endonuclease VII, and subsequent removal of the recognized and cleaved erroneous ligation products, for example by exonuclease I), then the fraction of the "correct" clones is enhanced to 27.6%. Considering that in this experiment λ = 0,59 ± 0.05, then the corrected fraction of "correct" clones is even enhanced to about 47% (compare the diagram of Fig. 2, column 4).

The removal of mismatches and the subsequent PCR can be catalyzed by thermostable DNA polymerases with 3'-5'-exonuclease activity, so-called "proof-reading" polymerases, for example, Vent-DNA-polymerase (NEB), in a single step (compare the diagram of Fig. 1, column C4). In comparison to the correction demonstrated by 4, there is a marked advantage, when the enzyme binding and cleaving step is done for longer periods (for example 24 hours as demonstrated in columns 9). After 24 hours, almost all clones that contain a DNA fragment of the required size ($\lambda$ =0,22) are "correct" (84%$\pm$11).

## Claims

1. A process for reducing the number of mismatches in a population of double-stranded polynucleotides, that process comprising the following steps:

   a) cleaving the double-stranded polynucleotide in the vicinity of a mismatch enzymatically, then
   b) removing the previously mismatching nucleotides from both strands of the double-stranded polynucleotide enzymatically,
   c) filling the resulting gaps by separating the strands and allowing them to anneal to form overlapping templates, followed by enzymatic polymerization for completing a double strand with filled gaps.

2. The process of claim 1, comprising an additional step of amplifying the resulting double-stranded polynucleotides.

3. The process of claim 2, wherein the polynucleotide is a DNA or RNA polynucleotide.

4. The process according to claim 3, wherein the polynucleotide is a DNA polynucleotide.

5. The process of any one of claims 1 to 4, wherein the enzyme in step a) and/or b) is selected from the group consisting of T4 Endonuclease VII, T7 Endonuclease I, *E. coli* Endonuclease V, *Aspergillus oryzae* S1 nuclease, *Ustilago maydis* DNAse I, *E. coli* Mut Y, *E. coli* Exonucleasenuclease I, *Vent-DNA-polymerase,* Pfu DNA polymerase, and mung bean nuclease.

6. The process of any one of claims 1 to 4, wherein the enzyme in step a) is T4 Endonuclease VII and the enzyme in step b) is *E. coli* Exonuclease I.

7. The process according to any one of claims 2 to 6, wherein the amplification is an overlap-extension PCR amplification.

8. The process of any one of claims 1 to 5, wherein a polymerase used in step b) and/or step c) is a proofreading polymerase.

9. The process of any one of claims 1 to 6, wherein the polymerase used in step b) or step c) is Vent-DNA-Polymerase.

10. The process of any one of claims 1 to 8, wherein the enzyme used in step a) is T4 Endonuclease VII, the enzyme used in step b) and step c) is Vent-DNA-Polymerase.

11. A process of producing double-stranded polynucleotides having a reduced number of mismatches in their double strands, said process comprising the following steps:

    a) providing *n* 5'-phosphorylated linkable oligonucleotides designed to form a first strand and *m* oligonucleotide (s) corresponding to the complementary strand, wherein each oligonucleotide of the complementary strand overlaps with at least two oligonucleotides of the first strand, wherein *n* is an integer from 2 to 100 and *m* is between *n* -1 and *n* + 1.
    b) allowing the oligonucleotides of the first strand to anneal to those of the second strand
    c) linking the oligonucleotides of both strands to produce a double-stranded polynucleotide
    d) reducing the number of mismatches in the population by subjecting the double-stranded polynucleotide of step c) to a process according to any one of claims 1-10.

12. Use of an enzyme for cleaving and/or removing cleaved mismatches in double stranded polynucleotides in a process according to any one of claims 1 to 11.

**13.** Use of an enzyme according to claim 12, wherein the enzyme is selected from the group consisting of T4 Endonuclease VII, T7 Endonuclease I, *E. coli* Endonuclease *V, Aspergillus oryzae* S1 nuclease, *Ustilago maydis* DNAseI, *E. coli* Mut Y, *E. coli* Exonuclease I, *Vent-DNA-polymerase,* Pfu DNA polymerase, and mung bean nuclease.

**14.** A kit of parts, comprising at least one enzyme for cleaving mismatches of double stranded polynucleotides, and at least one enzyme for enzymatic removal of cleaved mismatches in said double stranded polynucleotides.

**15.** The kit according to claim 14, additionally comprising at least one enzyme capable of performing a PCR (polymerase chain reaction).

Fig. 1

principle of mismatch removal step:

mismatch cleaving enzyme T4 Endonuclease VII

melting of short overhangs at 37°C generates single stranded overhangs

single strand specific exonuclease (E. coli Exonuclease I)

Fig. 2

| | single strand synthesis (ss) | double strand synthesis (ds) | ds 4h endo VII Taq-Pol | ds 4h endo7/exol (a) Taq-Pol | ds 4h endo7/exol (b) Taq-Pol | ds 4h endo7 Vent-Pol | ds 24h endo7/exol (a) Taq-Pol | ds 24h endo7/exol (b) Taq-Pol | ds 24h endo VII Vent-Pol |
|---|---|---|---|---|---|---|---|---|---|
| relative frequency CAMr/CAT | 4,2% | 2,4% | 2,9% | 46,8% | 11,4% | 34,2% | 13,0% | 22,6% | 83,6% |
| error | ± 0 | ± 0 | ± 0 | ± 4 | ±1 | ±8 | ±8 | ±13 | ±11 |
| | | | | | | | | | |

*the calculation of the error bars is taken from the results of Table 1.

Fig. 3.1 – 3.5

1. OLIGONUCLEOTIDEMIX, PHOSPHORYLATED

2. LIGATION REACTION

3. MISMATCH RECOGNITION AND CLEAVAGE OF BOTH STRANDS (ENDONUCLEASE)

4. MISMATCH REMOVAL BY ENDONUCLEASE REACTION

5. NEW OLIGO PAIRING AND EXTENSION: OVERLAP EXTENSION

IN COMBINATION WITH A PCR USING TERMINAL PRIMERS

CORRECTED END PRODUCT, CLONABLE

24

**European Patent Office**

## EUROPEAN SEARCH REPORT

Application Number

EP 04 00 5913

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01/31060 A (GRANT STRUAN ; BLONDAL THORARINN (IS); DECODE GENETICS EHF (IS)) 3 May 2001 (2001-05-03) * page 1, line 25 - page 3, line 2; figure 1 * | 14,15 | C12N15/10 |
| A | US 2003/157682 A1 (FITZMAURICE WAYNE P ET AL) 21 August 2003 (2003-08-21) * paragraph [0097]; examples 1-8 * | 1-15 | |
| A | WO 03/072832 A (WISCONSIN ALUMNI RES FOUND) 4 September 2003 (2003-09-04) * paragraph [0008] - paragraph [0010] * * paragraph [0022] - paragraph [0026]; figures 2-4; examples * | 1-15 | |
| A | WO 03/048395 A (ZYMOGENETICS INC) 12 June 2003 (2003-06-12) * page 12, line 18 - page 17, line 24; examples 1-3,6 * | 1-15 | |
| A | MASHAL R D ET AL: "DETECTION OF MUTATIONS BY CLEAVAGE OF DNA HETERODUPLEXES WITH BACTERIOPHAGE RESOLVASES" NATURE GENETICS, NEW YORK, NY, US, vol. 9, 1 February 1995 (1995-02-01), pages 177-183, XP000600255 ISSN: 1061-4036 * the whole document * | | TECHNICAL FIELDS SEARCHED (Int.Cl.7) C12N |
| A | YOUIL R ET AL: "SCREENING FOR MUTATIONS BY ENZYME MISMATCH CLEAVAGE WITH T4 ENDONUCLEASE VII" PROCEEDINGS OF THE NATIONAL ACADEMY OF SCIENCES OF USA, NATIONAL ACADEMY OF SCIENCE. WASHINGTON, US, vol. 92, 1995, pages 87-91, XP002011671 ISSN: 0027-8424 * the whole document * | | |

The present search report has been drawn up for all claims

| Place of search | Date of completion of the search | Examiner |
|---|---|---|
| Munich | 10 September 2004 | Steffen, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 04 00 5913

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2004

| Patent document cited in search report | | Publication date | Patent family member(s) | | Publication date |
|---|---|---|---|---|---|
| WO 0131060 | A | 03-05-2001 | US | 6524794 B1 | 25-02-2003 |
| | | | AU | 1048501 A | 08-05-2001 |
| | | | CA | 2388921 A1 | 03-05-2001 |
| | | | CN | 1382222 T | 27-11-2002 |
| | | | EP | 1224329 A2 | 24-07-2002 |
| | | | WO | 0131060 A2 | 03-05-2001 |
| | | | JP | 2003512083 T | 02-04-2003 |
| US 2003157682 | A1 | 21-08-2003 | US | 2003148315 A1 | 07-08-2003 |
| | | | US | 2003157495 A1 | 21-08-2003 |
| | | | WO | 03066809 A2 | 14-08-2003 |
| WO 03072832 | A | 04-09-2003 | WO | 03072832 A1 | 04-09-2003 |
| | | | US | 2004132029 A1 | 08-07-2004 |
| WO 03048395 | A | 12-06-2003 | WO | 03048395 A1 | 12-06-2003 |
| | | | US | 2003143605 A1 | 31-07-2003 |

EPO FORM P0459

For more details about this annex : see Official Journal of the European Patent Office, No. 12/82

SEQUENCE LISTING

<110> University of Regensburg

<120> Process for reducing the number of mismatches in double stranded polynucleotides

>130> EP27934

<160> 36

<170> PatentIn Ver. 2.1

<210> 1
<211> 18
<212> DNA
<213> Unbekannt

<223> CATF01

<400> 1

ccg ctc gag atg gcc gag     18


<210> 2
<211> 44
<212> DNA
<213> Unbekannt

<223> CATF02

<400> 2

aag aag atc acc ggc tac acc acc gtg gac atc tcc cag tgg ca     44


<210> 3
<211> 42
<212> DNA
<213> Unbekannt

<223> CATF03

<400> 3

ccg caa gga gca ctt cga ggc ctt cca gtc cgt cgc cca gtg     42


<210> 4
<211> 43
<212> DNA
<213> Unbekannt

<223> CATF04

<400> 4

cac cta caa cca gac cgt gca gct gga cat cac cgc ctt cct g     43


<210> 5
<211> 45

<212> DNA
<213> Unbekannt

<223> CATF05

<400> 5

aag acc gtg aag aag aac aag cac aag ttc tac ccc gcc ttc atc    45

<210> 6
<211> 41
<212> DNA
<213> Unbekannt

<223> CATF06

<400> 6

cac atc ctg gcc cgc ctg atg aac gcc cac ccc gag ttc cg    41

<210> 7
<211> 42
<212> DNA
<213> Unbekannt

<223> CATF07

<400> 7

cat ggc cat gaa gga cgg cga gct ggt gat ctg gga  ctc cgt    42

<210> 8
<211> 42
<212> DNA
<213> Unbekannt

<223> CATF08

<400> 8

cca ccc ctg cta cac cgt gtt cca cga gca gac cga gac ctt    42

<210> 9
<211> 42
<212> DNA
<213> Unbekannt

<223> CATF09

<400> 9

ctc ctc cct gtg gtc cga gta cca cga cga ctt ccg cca gtt    42

<210> 10
<211> 41
<212> DNA
<213> Unbekannt

<223> CATF10

<400> 10

cct gca cat cta ctc cca gga cgt cgc ctg cta cgg cga ga    41


<210> 11
<211> 45
<212> DNA
<213> Unbekannt

<223> CATF11

<400> 11

acc tgg cct act tcc cca agg gct tca tcg aga aca tgt tct tcg    45


<210> 12
<211> 43
<212> DNA
<213> Unbekannt

<223> CATF12

<400> 12

tgt ccg cca acc cct ggg tgt cct tca cct cct tcg acc tga a    43


<210> 13
<211> 43
<212> DNA
<213> Unbekannt

<223> CATF13

<400> 13

cgt ggc caa cat gga caa ctt ctt cgc ccc cgt gtt cac cat g    43


<210> 14
<211> 42
<212> DNA
<213> Unbekannt

<223> CATF14

<400> 14

ggc aag tac tac acc cag ggc gac aag gtg ctg atg ccc ctg    42


<210> 15
<211> 40
<212> DNA
<213> Unbekannt

<223> CATF15

<400> 15

gcc atc cag gtc cac cac gcc gtg tgc gac ggc ttc cac g    40

<210> 16
<211> 42
<212> DNA
<213> Unbekannt

<223> CATF16

<400> 16

tcg gcc gca tgc tga acg agc tgc agc agt act gcg acg agt    42

<210> 17
<211> 34
<212> DNA
<213> Unbekannt

<223> CATF17

<400> 17

ggc agg gcg gcg ccc tac gta tct aag gat ccc g    34

<210> 18
<211> 41
<212> DNA
<213> Unbekannt

<223> CATR01

<400> 18

gtg gtg tag ccg gtg atc ttc ttc tcg gcc atc tcg agc gg    41

<210> 19
<211> 42
<212> DNA
<213> Unbekannt

<223> CATR02

<400> 19

gcc tcg aag tgc tcc ttg cgg tgc cac tgg gag atg tcc acg    42

<210> 20
<211> 43
<212> DNA
<213> Unbekannt

<223> CATR03

<400> 20

ctg cac ggt ctg gtt gta ggt gca ctg ggc gac gga ctg gaa g    43

```
<210> 21
<211> 44
<212> DNA
<213> Unbekannt

<223> CATR04

<400> 21

tgc ttg ttc ttc ttc acg gtc ttc agg agg gcg gtg atg tcc ag      44


<210> 22
<211> 43
<212> DNA
<213> Unbekannt

<223> CATR05

<400> 22

cat cag gcg ggc cag gat gtg gat gaa ggc ggg gta gaa ctt g      43


<210> 23
<211> 41
<212> DNA
<213> Unbekannt

<223> CATR06

<400> 23

tcg ccg tcc ttc atg gcc atg cgg aac tcg ggg tgg gcg tt      41


<210> 24
<211> 42
<212> DNA
<213> Unbekannt

<223> CATR07

<400> 24

aac acg gtg tag cag ggg tgg acg gag tcc cag atc acc agc      42


<210> 25
<211> 42
<212> DNA
<213> Unbekannt

<223> CATR08

<400> 25

tac tcg gac cac agg cag gag aag gtc tcg gtc tgc tcg tgg      42


<210> 26
```

```
<211> 42
<212> DNA
<213> Unbekannt

<223> CATR09

<400> 26

tcc tgg gag tag atg tgc agg aac tgg cgg aag tcg tcg tgg     42


<210> 27
<211> 41
<212> DNA
<213> Unbekannt

<223> CATR10

<400> 27

cct tgg gga agt agg cca ggt tct cgc cgt agc agg cga cg     41


<210> 28
<211> 45
<212> DNA
<213> Unbekannt

<223> CATR11

<400> 28

aca ccc agg ggt tgg cga aca cga aga aca tgt tct cga tga agc     45


<210> 29
<211> 44
<212> DNA
<213> Unbekannt

<223> CATR12

<400> 29

gaa gtt gtc cat gtt ggc cac gtt cag gtc gaa gga ggt gaa ga     44


<210> 30
<211> 42
<212> DNA
<213> Unbekannt

<223> CATR13

<400> 30

gcc ctg ggt gta gta ctt gcc cat ggt gaa cac ggg ggc gaa     42


<210> 31
<211> 41
<212> DNA
<213> Unbekannt
```

<223> CATR14

<400> 31

gcg tgg tgg acc tgg atg gcc agg ggc atc agc acc ttg tc    41


<210> 32
<211> 41
<212> DNA
<213> Unbekannt

<223> CATR15

<400> 32

gct cgt tca gca tgc ggc cga cgt gga agc cgt cgc aca cg    41


<210> 33
<211> 40
<212> DNA
<213> Unbekannt

<223> CATR16

<400> 33

cgt agg gcg ccg ccc tgc cac tcg tcg cag tac tgc tgc a    40


<210> 34
<211> 30
<212> DNA
<213> Unbekannt

<223> CATR17

<400> 34

cgg gat cct tag gcg ccg ccc tgc cac tcg    30


<210> 35
<211> 18
<212> DNA
<213> Unbekannt

<223> CATF01

<400> 35

ccg ctc gag atg gcc gag    18


<210> 36
<211> 27
<212> DNA
<213> Unbekannt

<223> CATR17

```
<400>36

cgg gat cct tag gcg ccg ccc tgc cac    27
```